# EUROPÄISCHE PATENTSCHRIFT

(11) **EP 1 420 707 B1**
(45) Veröffentlichungstag und Bekanntmachung des Hinweises auf die Patenterteilung: **23.02.2005**
(21) Anmeldenummer: 03766058.6
(22) Anmeldetag: 21.07.2003
(51) Int. Cl.: A61B 17/58, A61F 2/46

(54) **CHIRURGISCHES IMPLANTAT ODER INSTRUMENT MIT SICHERUNGSSCHRAUBE**
PROSTHESIS OR SURGICAL INSTRUMENT EQUIPPED WITH A FIXING SCREW
PROTHESE OU INSTRUMENT CHIRURGICAL DOTE D'UNE VIS DE FIXATION

(30) Priorität: 16.08.2002 DE 20212600 U
(43) Veröffentlichungstag der Anmeldung: 26.05.2004
(73) Patentinhaber: WALDEMAR LINK GmbH & Co. KG, 22339 Hamburg (DE)
(72) Erfinder: KELLER, Arnold, 23863 Kayhude (DE)
(74) Vertreter: Glawe, Delfs, Moll & Partner
(86) Internationale Anmeldenummer: PCT/EP2003/007942
(87) Internationale Veröffentlichungsnummer: WO 2004/017846

(56) Entgegenhaltungen:
- EP-A- 0 589 325
- EP-A- 1 031 332
- DE-A- 19 501 882
- US-A- 6 099 569

## Beschreibung

Die Erfindung betrifft ein chirurgisches Implantat oder Instrument mit Sicherungsschraube.

Bei chirurgischen Implantaten oder Instrumenten stellt sich mitunter die Aufgabe, zwei gegeneinander verschiebbar geführte Teile gegen Verschiebung durch eine Schraube zu sichern. Es ist bekannt, dies dadurch zu bewerkstelligen, daß eine Sicherungsschraube durch eine Durchgangsbohrung im oberen der beiden Teile in eine Gewindebohrung im unteren der beiden Teile geschraubt wird, bis sie mit ihrem Kopf fest auf der Oberseite des oberen der beiden Teile sitzt. Dies hat den Nachteil, daß die Schraube verloren gehen kann, was im unübersichtlichen Operationsfeld folgenschwer sein kann.

Es ist bekannt (EP-A-589325 (Basis für den Oberbegriff des Anspruchs 1), EP-A-1031332), dies dadurch zu vermeiden, daß eine Schraube verwendet wird, die kopflos ausgebildet ist und mit ihrer ganzen Länge in der Gewindebohrung (jedenfalls unterhalb der Unterfläche des oberen der beiden Teile) untergebracht werden kann. Sie weist einen oberen, dünneren Absatz auf, der eine Schraubendrehervertiefung enthält. Der Durchmesser der Durchgangsöffnung ist geringer als der des Schraubengewindes aber größer als der des Schraubenansatzes. Bevor die Teile zusammengefügt werden, schraubt man die Schraube in die Gewindebohrung so tief ein, daß sie ganz darin verschwindet oder jedenfalls so tief liegt, daß der obere der beiden Teile darüber hinweggleiten kann. Danach wird mit dem Schraubendreher durch die Durchgangsöffnung des oberen Teils hindurch die Schraube hochgeschraubt, bis ihr Ansatz in der Durchgangsöffnung liegt. Die beiden Teile sind nun gegen relative Verschiebung gesichert, soferndie Schraube hinreichend festgedreht ist. Wenn sich die Schraube jedoch lockert, kann es geschehen, daß sie allmählich so weit nach unten ausweicht, daß die Sicherungswirkung gefährdet wird.

Der Erfindung liegt daher die Aufgbe zugrunde, eine weitergehende Sicherung gegen ein Zurückweichen der Sicherungsschraube in die Gewindebohrung hinein zu erreichen. Erfindungsgemäß gelingt dies dadurch, daß sie vom unteren Ende her mit einer elastischen Kraft, beispielsweise einer Feder, beaufschlagt wird.

Die Begriffe "oben" und "unten", die in diesem Text und in den Ansprüchen verwendet werden, sind nicht im Sinne geodätischer Höhenlage zu deuten, sondern bezeichnen die Lage relativ zum Betrachter, der den Zugang zur Sicherungsschraube sucht. Der untere Teil ist, allgemeiner gesprochen, der vom oberen Teil abgedeckte Teil.

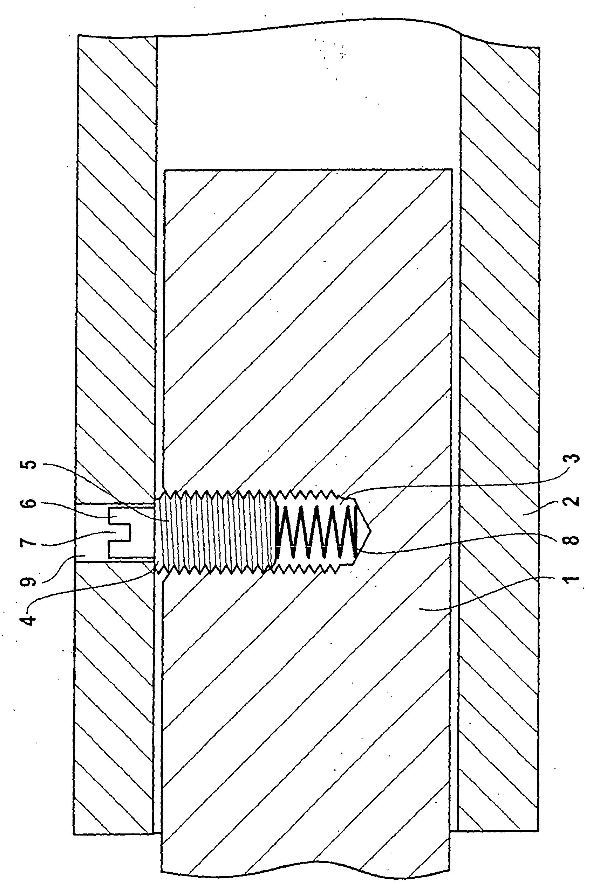

Die Erfindung wird im folgenden näher unter Bezugnahme auf die Zeichnung erläutert, die ein vorteilhaftes Ausführungsbeispiel in einer Schnittdarstellung veranschaulicht.

Ein Bolzen 1 ist mit einer Hülse 2 zu verbinden, die mit wenig größerem Durchmesser dem Bolzen übergeschoben wird. Ihre zusammenwirkenden Oberflächen bewirken eine Führung, die eine Relativbewegung zwischen den beiden Teilen nur parallel zu ihrer Längsachse erlaubt.

Der Bolzen 1 enthält eine quer zu seiner Längsachse verlaufende Gewindebohrung 3. Darin sitzt die Gewindeschraube 4, die einen Gewindeabschnitt 5 und einen oberen Ansatz 6 aufweist, der einen Schlitz oder Innensechskant 7 für die Verbindung mit einem Schraubendreher enthält. Der Ansatz 6 hat einen geringeren Durchmesser als der Gewindeabschnitt 5. Zwischen dem Grund der Gewindebohrung 3 und dem Boden der Schraube 4 befindet sich eine Druckfeder 8. Die Gewindebohrung 3 hat eine Länge, die - ohne Berücksichtigung der für die Aufnahme der komprimierten Feder 8 benötigten Länge, größer ist als diejenige der Schraube 4. Die Durchgangsbohrung 9 in Teil 2 hat einen Durchmesser, der geringer ist als der des Gewindeabschnitts 5 und größer als der des Ansatzes 6 der Schraube 4.

Vor der Montage wird die Schraube 4 gänzlich in die Gewindebohrung 3 versenkt. Mindestens darf sie nicht weiter daraus hervorragen als es dem Spiel zwischen den einander gegenüberstehenden Oberflächen der Teile 1 und 2 entspricht. Danach werden die Teile 1 und 2 zusammengeschoben, bis die Durchgangsöffnung 9 über der Schraube 4 liegt. Dann wird mittels eines Schraubendrehers die Schraube 4 soweit herausgeschraubt, bis ihr Ansatz 6 innerhalb der Durchgangsbohrung 9 liegt. Sie kann gewünschtenfalls festgedreht werden, bis die Schulter ihres Gewindeabschnitts 5 hinreichend stramm an dem Rand der Durchgangsbohrung 9 anliegt.

Letzteres ist nicht unbedingt erforderlich, da die Feder 8 vorhanden ist. Diese drängt nämlich die Schraube 4 nach oben, so daß sie nicht durch das Einwirken zufälliger Kräfte in die Gewindebohrung 3 zurücksinken kann.

Es versteht sich, daß die als Spiralfeder dargestellte Feder 8 durch andere Federmittel ersetzt werden kann, beispielsweise durch einen Schaumpfropfen aus elastischem Kunststoff.

## Patentansprüche

1. Chirurgisches Implantat oder Instrument mit zwei übereinander verschiebbar geführten Teilen (1, 2), von denen der untere (1) eine Gewindebohrung (3) und der obere (2) eine Durchgangsbohrung (9) zur Aufnahme einer Sicherungsschraube (4) enthalten, die kopflos ausgebildet ist und in voller Länge unterhalb des oberen Teils (2) in der Gewindebohrung (3) aufgenommen werden kann und am oberen Ende einen mit einer Schraubendrehervertiefung (7) versehenen Ansatz aufweist, der dünner ist als ihr Gewindeteil (5), wobei der Durchmesser der Durchgangsbohrung (9) geringer als der des Gewindeteils und größer als der des Ansatzes (6) ist, **dadurch gekennzeichnet, daß** eine elastische Einrichtung (8) zum Drängen der Sicherungsschraube (4) in die Sicherungsstellung vorgesehen ist.

2. Implantat oder Instrument nach Anspruch 1, **dadurch gekennzeichnet, daß** die Gewindebohrung (3) eine Sackbohrung ist.

3. Implantat oder Instrument nach Anspruch 1 oder 2, **dadurch gekennzeichnet, daß** die Sicherungsschraube (4) und die Gewindebohrung (3) zusammenwirkende Anschläge gegen eine Entfernung der Sicherungsschraube (4) aus der Gewindebohrung (3) nach unten aufweisen.

## Claims

1. A surgical implant or instrument having parts (1,2) guided one above the other, the lower part (1) of which comprises a threaded bore (3) and the upper part (2) of which comprises a through-bore (9) to receive a securing screw (4) which is of headless design and the full length of which can be accommodated below the upper part (2) in the threaded bore (3) and has at its upper end an extension which is provided with a recess (7) for a screwdriver and which is thinner than its threaded portion (5), wherein the diameter of the through-bore (9) is smaller than that of the threaded portion and larger than that of the extension (6), **characterised in that** an elastic means (8) is provided to urge the securing screw (4) into the securing position.

2. An implant or instrument according to Claim 1, **characterised in that** the threaded bore (3) is a blind bore.

3. An implant or instrument according to Claim 1 or 2, **characterised in that** the securing screw (4) and the threaded bore (3) have co-operating abutments to prevent the securing screw (4) from being removed downwardly from the threaded bore (3).

## Revendications

1. Prothèse ou instrument chirurgical comportant deux parties (1, 2) guidées de manière coulissante l'une au-dessus de l'autre, dont la partie inférieure (1) contient un trou taraudé (3) et la partie supérieure (2) un trou débouchant (9) destiné à recevoir une vis de blocage (4) qui est réalisée sans tête et peut être reçue sur toute sa longueur au-dessous de la partie supérieure (2), dans le trou taraudé (3), et qui présente à l'extrémité supérieure un appendice plus mince que sa partie filetée (5), pourvu d'un creux pour tournevis (7), le diamètre du trou débouchant (9) étant inférieur à celui de la partie filetée et supérieur à celui de l'appendice (6), **caractérisé en ce qu'**il est prévu un dispositif élastique (8) pour presser la vis de blocage (4) en position de blocage.

2. Prothèse ou instrument selon la revendication 1, **caractérisé en ce que** le trou taraudé (3) est un trou borgne.

3. Prothèse ou instrument selon la revendication 1 ou 2, **caractérisé en ce que** la vis de blocage (4) et le trou taraudé (3) présentent des butées coopérantes pour empêcher que la vis de blocage (4) ne soit enlevée vers le bas du trou taraudé (3).
